(19) Europäisches Patentamt
European Patent Office
Office européen des brevets

(11) **EP 3 849 586 B1**

(12) **EUROPEAN PATENT SPECIFICATION**

(45) Date of publication and mention
of the grant of the patent:
**11.03.2026 Bulletin 2026/11**

(21) Application number: **19783695.0**

(22) Date of filing: **14.09.2019**

(51) International Patent Classification (IPC):
*A61K 38/16* (2006.01)    *C07K 14/47* (2006.01)
*A61P 19/02* (2006.01)    *A61P 29/00* (2006.01)

(52) Cooperative Patent Classification (CPC):
**A61K 38/16; A61P 19/02; A61P 29/00;
C07K 14/4726**

(86) International application number:
**PCT/IB2019/057757**

(87) International publication number:
**WO 2020/074984 (16.04.2020 Gazette 2020/16)**

(54) **SCLEROTIUM ROLFSII LECTIN FOR TREATMENT OF INFLAMMATORY DISEASES**

SCLEROTIUM ROLFSII LECTIN ZUR BEHANDLUNG VON ENTZÜNDLICHEN ERKRANKUNGEN

LECTINE DE SCLEROTIUM ROLFSII POUR LE TRAITEMENT DE MALADIES INFLAMMATOIRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **16.09.2018 IN 201821009667**

(43) Date of publication of application:
**21.07.2021 Bulletin 2021/29**

(73) Proprietor: **Unichem Laboratories Ltd
Maharashtra 400067 (IN)**

(72) Inventors:
• **SATHE, Dhananjay**
**Thane, Maharashtra 400601 (IN)**
• **KUMAR, Sudeep**
**Ahmedabad, Gujarat 380058 (IN)**
• **KATDARE, Mamata**
**Pune, Maharashtra 411038 (IN)**

(74) Representative: **Marks & Clerk LLP
15 Fetter Lane
London EC4A 1BW (GB)**

(56) References cited:
WO-A1-00/57897        WO-A1-00/57897
WO-A1-2010/050369     WO-A1-2010/050369
WO-A2-2010/095143     WO-A2-2010/095143
GB-A- 2 270 626       GB-A- 2 270 626

Remarks:
The complete document including Reference Table(s) and the Sequence Listing(s) can be downloaded from the EPO website

## Description

### Cross Reference to Related Applications

This application claims the benefit of Indian Provisional Application No. 201821009667 filed on Sep 16, 2018.

### Field of invention

The present invention relates to the use of lectin proteins for the prevention, treatment and cure of inflammation, including inflammatory disease.

### Background of invention

Inflammation contributes in protecting the injured tissue from further infection by triggering a multifactorial network of chemical signals which initiate the healing process. This involves the activation and migration of important blood cells (neutrophils, monocytes and eosinophils) to the site of damage. The cells (macrophages, mast cells, endothelial cells and Schwann cells) secrete cytokines and specific growth factors required for nerve regeneration and eventually results in reconstitution of the normal microenvironment.

Inflammation associated with wound healing (normal inflammation) is usually self-limiting; however, dysregulation of any of the converging factors can lead to abnormalities and ultimately pathogenesis characterized by acute or chronic inflammation. Chronic inflammation is long-term inflammation and can last for several months and even years, whereas acute inflammation is one that starts rapidly and becomes severe in a short span of time. Chronic inflammations include Psoriasis/psoriatic arthritis, Rheumatoid arthritis (RA), active hepatitis, asthma, and inflammatory bowel disease, which need to be well managed and treated well in time. The damages caused by chronic inflammations are irreversible and highly painful.

At present the medications used to treat some of the inflammatory diseases vary depending on the type of disease. Commonly used medications include:

Analgesics help reduce pain, but have no effect on inflammation. Examples include acetaminophen (Tylenol, others), tramadol (Ultram, Ultracet, others) and narcotics containing oxycodone (Percocet, Oxycontin, others) or hydrocodone (Norco, Vicoprofen, others). Nonsteroidal anti-inflammatory drugs (NSAIDs) reduce both pain and inflammation. NSAIDs include ibuprofen (Advil, Motrin IB, others) and naproxen sodium (Aleve). Some counterirritant varieties of creams and ointments contain menthol or capsaicin. Disease-modifying antirheumatic drugs (DMARDs) are often used to treat rheumatoid arthritis. DMARDs slow or stop the immune system from attacking joints. Examples include methotrexate (Trexall) and hydroxychloroquine (Plaquenil). A new subcategory of DMARDs known as "JAK inhibitors" block the Janus kinase, or JAK, pathways, which are involved in the body's immune response. Tofacitinib belongs to this class. Unlike biologics, it can be taken by mouth. Typically used in conjunction with DMARDs, biologic response modifiers are genetically engineered drugs that target various protein molecules that are involved in the immune response. They are BIT-cells inhibitors, selective costimulation modulators. Examples include etanercept (Enbrel), certolizumab (Cimzia), Rituximab (Rituxan) and infliximab (Remicade). Corticosteroids such as prednisone and cortisone, reduces inflammation and suppresses the immune system.

The medications can be accompanied by therapy (physical, hot and cold), weight loss, braces, exercise, TENS and surgery. However, each of the medications mentioned above has side effects of its own. Therefore, biologics remain the choice. These drugs are a subset of DMARDs. Biologics may work more quickly than traditional DMARDs, and are injected or given by infusion. Because they target specific steps in the inflammatory process, they don't wipe out the entire immune response as some other inflammatory disease treatments do. In many people with inflammatory diseases, a biologic can slow, modify or stop the disease - even when other treatments haven't helped much. Today there are very few approved biologics for the treatment of inflammatory diseases. They are highly costly and are scarcely available due to less production and high demand. Thus there is an unmet need to provide an option for the treatment of inflammatory diseases, wherein the option is highly specific towards its action, is cheap and effectively accomplishes the market needs.

WO 2010/050369A1 describes a prophylactic agent comprising a lectin for dental caries. GB2270626A describes the use of lectins for the treatment of skin diseases.

WO00/57897A1 describes the use of a lectin and lectin conjugates for modulation of C-fibre activity.

WO 2010/095143A2 describes recombinant lectins having anti-tumour activity.

### Object of invention

Therefore an object of the present invention is to provide a highly specific and effective biologic for the treatment of inflammatory diseases.

Yet another object of the present disclosure is to provide an economical and effective method of treatment of inflammatory diseases.

Another object of the present invention is to provide recombinant protein for the treatment of inflammatory diseases.

Yet another object of the present present disclosure is to provide a method of treatment of inflammatory diseases using recombinant protein.

Yet another object of the present disclosure is to provide a method to cure or reduce the effect of inflammatory diseases in subject suffering from the diseases.

## Summary of invention

According to a first aspect of the present invention, there is provided a lectin protein for use in the treatment or prevention of inflammation in a subject in need thereof, wherein the lectin protein is recombinant, is derived from *Sclerotium rolfsii,* and comprises an amino acid sequence of SEQ ID NO: 2.

Preferably, treatment or prevention of inflammation comprises a) inhibiting the secretion of inflammatory cytokines; and/or b) reducing C-reactive protein (CRP) levels in blood; and/or c) stimulating ALP activity and/or collagen levels.

In some embodiments, the subject has a chronic inflammatory disease.

The chronic inflammatory disease may be psoriasis, psoriatic arthritis, rheumatoid arthritis (RA), hepatitis, asthma, inflammatory bowel disease (including crohn's disease, colitis and irritable bowel syndrome (IBS)), temporal artheritis, atopic dermatitis, systemic lupus, multiple sclerosis or sarcoidosis.

Preferably, the chronic inflammatory disease is rheumatoid arthritis (RA).

In some embodiments, the subject has acute inflammation. The acute inflammation may be caused by an allergic reaction, an infection or trauma.

Preferably, the treatment comprises curing the inflammation or the chronic inflammatory disease, or reducing symptoms associated with the inflammation.

Preferably, the prevention comprises preventing the onset of the inflammation, or preventing or slowing the progression of a chronic inflammatory disease.

Conveniently, treatment or prevention comprises administering the lectin protein at a dose of 1 to 50 mg/kg.

Lectins are highly specific carbohydrate-binding proteins, macromolecules that are highly specific for sugar moieties of other molecules. Lectins perform recognition on the cellular and molecular level and play numerous roles in biological recognition phenomena involving cells, carbohydrates, and proteins. They are divalent or polyvalent carbohydrate-binding proteins that bind and precipitate glycoproteins and agglutinate red blood cells. Lectins found in animals are most often found to aid in cell interactions, while plant lectins are known to ward off potential predators or pathogens.

Purified lectins are important in a clinical setting because they are used for blood typing. Some of the glycolipids and glycoproteins on an individual's red blood cells can be identified by lectins. Many lectins are used as biomarkers indicating early detection of malignant growth or as autophagy inducers while other lectins also show the ability to inhibit cancerous growth through apoptosis. Due to unregulated cell proliferation, some of the carbohydrate moieties are expressed as an antigen on cancerous cells. Lectins are used as a drug delivery agent in cancer therapy because they bind specifically to the malignant tumours. Further since the lectins also modulate cancer associated pathways they have potential as cancer diagnostic and therapeutic agents.

There are several antigens to which lectins bind and which have been characterised on the cancer cell surface; most of the antigens are specific for a particular type of cancer and lectin-binding to these antigens can result in inhibition of cancerous growth through inducing apoptosis in the cancerous cells. Currently, most commercially available lectins are from plants and other eukaryotes.

*Sclerotium rolfsii* lectin (SRL) is a lectin that has been isolated from the sclerotial bodies of the soil-borne phytopathogenic fungus S. *rolfsii.* SRL has been shown to bind to human colon cancer, ovarian cancer and leukemic cells.

Surprisingly, the present inventors have now found that lectins are useful in the treatment of inflammation.

According to a second aspect of the invention, there is provided a pharmaceutical composition comprising a lectin protein and a pharmaceutically acceptable diluent or excipient, wherein the lectin protein is recombinant, is derived from *Sclerotium rolfsii,* and comprises an amino acid sequence of SEQ ID NO:2 for use in a method of treatment or prevention of inflammation in a subject, the method comprising administering the pharmaceutical composition to the subject.

Preferably, the inflammation is an inflammatory disease. The inflammatory disease may be arthritis. In some embodiments, the method comprises administering the lectin protein at a dose of 1 to 50 mg/kg.

The lectin protein for use according to the invention may be administered to the subject by injecting the solution or suspension intravenously, intramuscularly, intraperitoneally, subcutaneously, intradermally, by depot injection, intrathecally, transdermally, sublingually or by oral, topical or inhalation methods.

The term "lectin" as used herein refers to a carbohydrate-binding protein.

The term "protein" as used herein refers to a polymer of amino acid residues.

The term "amino acid" as used herein refers to naturally occurring and synthetic amino acids, as well as amino acid analogues and amino acid mimetics that have a function that is similar to the naturally occurring amino acids. Naturally occurring amino acids are those encoded by the genetic code and include the proteinogenic amino acids (i.e. the amino acids which are incorporated biosynthetically into proteins during translation in cells). Naturally occurring amino acids also include those modified after translation in cells. Synthetic amino acids include non-canonical amino acids such as selenocysteine and pyrrolysine. Typically synthetic amino acids are not proteinogenic amino acids.

It is understood that amino acids can be grouped according to different biochemical properties. Examples include: the polar amino acids, the non-polar amino acids, the acidic amino acids and the basic amino acids. In one embodiment, the amino acid used for the amino acid modification is at least one selected from the group consisting of, but not limited to: polar, non-polar, acidic, basic, selenocysteine, pyrrolysine and non-canonical.

The terms "homology" or "homologous" as used herein refer to two or more referenced entities that share at least partial identity over a given region or portion. Areas, regions or domains of homology or identity refer to a portion of two or more referenced entities that share homology or are the same. Thus, where two sequences are identical over one or more sequence regions they share identity in these regions. Substantial homology refers to a molecule that is structurally or functionally conserved such that it has or is predicted to have at least partial structure or function of one or more of the structures or functions (e.g., a biological function or activity) of the reference molecule, or a relevant/corresponding region or portion of the reference molecule to which it shares homology.

In one embodiment, the percentage "homology" between two sequences is determined using the BLASTP algorithm with default parameters (Altschul et al. Nucleic Acids Res. 1997 Sep 1;25(17):3389-402). In particular, the BLAST algorithm can be accessed on the internet using the URL: https://blast.ncbi.nlm.nih.gov/Blast.cgi. In an alternative embodiment, for global sequence alignments, percentage homology between two sequences is determined using the EMBOSS Needle algorithm using default parameters. In particular, the EMBOSS Needle algorithm can be accessed on the internet using the URL: https://www.ebi.ac. uk/Tools/psa/emboss_needle/.

Unless otherwise indicated, the term "homology" is used interchangeably with the term "sequence identity" in the present specification.

Inflammation typically presents symptoms of swollen, red, hot patches on your body. However, inflammation may occur without any obvious symptoms. Inflammation can be detected by measuring high-sensitivity C-reactive protein and white blood cell count. These can be measured with a blood test.

C-reactive protein (CRP), a protein in the blood, is one of the best indicators of inflammation as levels of CRP rise as inflammation increases. In some embodiments, the term "inflammation" means that a subject has blood with a concentration of C- reactive protein of at least 1.0 mg/L, at least 3.0 mg/L, or at least 10.0 mg/L.

A high white blood cell can indicate infection, stress, inflammation, trauma, allergy, or certain diseases.

The normal range of white blood cell count for adults is generally 4,000-10,500 cells per mL of blood. Above 10,500 is considered to be high. In some embodiments, the term "inflammation" means that a subject has a white blood cell count of at least 10,500 cells per mL

Insulin is a hormone produced and stored by the pancreas, which helps to regulate blood glucose levels. Insulin resistance is the decreased ability to respond to the effects of insulin. As a result, the body produces additional amounts of insulin which increases inflammatory processes within the body. The clinical range is 2.6-24.9 $\mu$IU/mL; the optimal range is 1.0-5.0 $\mu$IU/mL.

In some embodiments, the term "inflammation" means that a subject has an insulin level of at least 5.0 $\mu$IU/mL or at least 24.9 $\mu$IU/mL.

Hemoglobin A1C (HbAlc) provides the average amount of glucose in the blood, or blood sugar, over the previous 3 months (the life of red blood cells is 3 months). Hemoglobin Al c (or glycated hemoglobin) is formed in the blood when glucose attaches to hemoglobin, the protein in red blood cells that carries oxygen. The higher the level of glucose in the blood, the more glycated hemoglobin is formed. Chronically elevated blood sugar reacts with enzymes and other protein molecules to create Advanced Glycation End Products (AGEs). AGEs are highly inflammatory and damage tissue throughout the body. The result is neurological and cardiovascular complications which are common with diabetes. The clinical range is 4.8%-5.6%; the optimal range is 4.5%-5.2%. HbAlc levels are typically measured as a percentage of glycated hemoglobin compared to unglycated hemoglobin or as a dimensionless unit.

In some embodiments, the term "inflammation" means that a subject has a HbAlc level of at least 5.2%, or at least 5.6%.

Ferritin is a blood cell protein that stores iron. Low levels of ferritin indicate iron deficiency which causes anemia, a reduction in the number of red blood cells. Like CRP, ferritin is an acute phase reactant. This makes a ferritin test useful in detecting a chronic disease process. Elevated levels of ferritin can indicate inflammation. The clinical range: 30-400 ng/mL; the optimal range is forfemales (10-300 ng/mL), and for males (23-336 ng/mL).

In some embodiments, the term "inflammation" means that a subject has a ferritin level of at least 200 ng/mL, at least 300 ng/mL, or at least 336 ng/mL.

Red Cell Distribution Width (RDW) is an excellent test to detect inflammation in the body. RDW is a "robust predictor" of the risk of mortality from all causes and bloodstream inflection. RDW reflects overall inflammation and oxidative stress. RDW is an expression of the variation in size of the red blood cells that make up the total population of red blood cells in an individual. The size of a blood vessel has a lot to do with the maturation of the blood cells and this is dependent upon methylating agents such folate and BI 2. The clinical range is 12.3-15.4%; the optimal range is 11.6-15%.

In some embodiments, the term "inflammation" means that a subject has a red blood distribution width of at least 15%, or at least 15.1%.

Rheumatoid factor is an antibody that acts against the blood component gamma globulin. The clinical range is 20 IU/mL or higher; the optimal range is 0-20 IU/mL

In some embodiments, the term "inflammation" means that a subject has a rheumatoid factor level of at least 20 IU/mL.

T cells and macrophages both secrete IL-6 as part of the inflammatory response, so elevated IL-6 in a subject can indicate systemic inflammation.

**Brief Description of the Accompanying Sequences**

- SEQ ID NO: 1: represents the native S. *rolfsii* lectin amino acid sequence.

- SEQ ID NO: 2: represents a variant of the S. *rolfsii* lectin amino acid sequence (reported as Rec-2 in WO 2010/095143).

- SEQ ID NO: 3: represents a variant of the S. *rolfsii* lectin amino acid sequence (reported as Rec-3 in WO 2010/095143).

- SEQ ID NO: 4: represents a variant of the S. *rolfsii* lectin amino acid sequence (reported in WO 2014/203261).

SEQ ID NO: 1 has the following sequence:

TYKITVRVYQTNPNAFFHPVEKTVWKYANGGTWTITDDQHVLTMGGSGTSG

TLRFHADNGESFTATFGVHNYKRWCDIVTNLAADETGMVINQQYYSQKNRE

EARERQLSNYEVKNAKGRNFEIVYTEAEGNDLHANLIIG

SEQ ID NO: 2 has the following sequence:

TYKITVRVYQTNP<u>D</u>AFFHPVEKTVWKYANGGTWTITDDQHVLTMGGSGTSG

TLRFHADNGESFTATFGVHNYKRWCDIVTNLAADETGMVINQQYYSQKNRE

EARERQLSNY<u>Q</u>VKNAKGRNF<u>Q</u>IVYTEAEGNDLHANLIIG

SEQ ID NO: 3 has the following sequence:

<u>V</u>YKITVRVYQTNP<u>D</u>AFFHPVEKTVWKYANGGTW<u>S</u>ITDDQHVLTMGGSGTSG

TLRFHADNGESFTATFGVHNYKRWCDIVTNLAADETGMVINQQYYSQKNRE

EARERQLSNY<u>Q</u>VKNAKGRNF<u>Q</u>IVYTEAEGNDLHANLIIG

SEQ ID NO: 4 has the following sequence:

VYKITVRVYQTNPDAFFHPVEKTVWKYADGGTWSITDDQHVLTMGGSGTSG

TLRFHADNGESFTATFGVHDYKRWCDIVTDLAADETGMVINQEYYSEKDRE

EARERQNSNYEVKDAKGRNFEIVYTEAEGNDLHADLIIG

Differences in the sequence of SEQ ID NOs: 2-4 compared with the sequence of SEQ ID NO: 1 have been underlined.

**Detailed Description of the invention**

Any references to methods of treatment in this description are to be interpreted as references to compounds, pharmaceutical compositions and medicaments comprising a lectin according to the present invention for use in those methods.

The present invention provides a lectin for use in the treatment or prevention of inflammation in a subject in need thereof.

The lectin for use according to the invention is a recombinant lectin having an amino acid sequence of SEQ ID NO: 2 which is derived from S. *rolfsii. By* "derived from" it will be understood that the lectin comprises an amino acid sequence which is similar to a native sequence.

SEQ ID NO: 2 has 98% homology with SEQ ID NO: 1. For reference, SEQ ID NO: 3 has 96% homology with SEQ ID NO: 1. SEQ ID NO: 4 has 91% homology with SEQ ID NO: 1.

In the present specification, the term "recombinant" means that lectin gene is transferred into a host cell (such as bacteria or yeast) and the gene is then expressed to produce the lectin. Methods for preparing recombinant proteins will be well-known to those skilled in the art. For example, a recombinant DNA molecule, such as a plasmid or viral vector, comprising a nucleic acid sequence encoding the lectin may be provided. The nucleic acid sequence may be operatively linked to a promoter which is capable of controlling expression of the lectin in a suitable host cell. The recombinant DNA molecule may be inserted into a suitable host cell using methods known in the art, for example by transformation. Suitable host cells include prokaryotic cells (e.g. E. *coli)* and both lower eukaryotic cells (e.g. yeast cells) as well as higher eukaryotic cells. The host cell can then be cultured under appropriate conditions, whereby the recombinant lectin is expressed. The recombinant lectin can thus be obtained by isolation as an expression product from the host cell. Recombinant proteins can be purified by conventional techniques known in the art, typically conventional chromatographic methods.

The lectin may have at least one anti-inflammatory activity.

In some embodiments, the lectin is capable of inhibiting or reducing the secretion of an inflammatory cytokine.

As is known in the art, cytokines are small proteins involved in cell signaling. Cytokines include chemokines, interferons, interleukins, lymphokines, and tumour necrosis factors (TNF). As used herein, "Interleukin" (IL) includes IL-1, IL-2, IL-6, IL-8, IL-10, IL-12, IL-13, IL-15, IL-17, IL-18, IL-1β, IL-17A, IL-17F, IL-22, IL-26, IL-23, IL-20, and IL-15.

The lectin may be capable of inhibiting or reducing the secretion of one or more inflammatory cytokines selected from IL-8, IL-6, TNF-α, IL-1-β, IFN-γ and GM-CSF.

The ability of a lectin to inhibit or reduce the secretion of inflammatory cytokines may be determined using standard techniques, including the methods described herein. For example, the level of an inflammatory cytokine secreted following an inflammatory stimulus (e.g. by IFN-γ or LPS) in the presence of the lectin can be determined and compared to the level of the cytokine secreted following the inflammatory stimulus in the absence of the lectin. Levels of cytokines in samples such as cell culture supernatants (for *in vitro* studies) or serum samples (for *in vivo* studies) can be determined using standard techniques such as ELISA

In some embodiments, the lectin is capable of reducing the secretion of an inflammatory cytokine by 5 to 50%, 10 to 40%, 15 to 35% or 20 to 30%.

In some embodiments the lectin is capable of reducing the level of C-reactive protein (CRP) levels in blood. The level of CRP in blood can be determined using standard techniques, such as those described herein. For example, the lectin may be capable of reducing the level of CRP in blood by at least 2%, at least 5% or at least 10% per 10 mg/kg of lectin administered to a subject, compared to a control subject to which no lectin has been administered.

In some embodiments the lectin is capable of stimulating (i.e. increasing) Alkaline Phosphatase (ALP) activity and/or collagen levels. ALP levels, for example in cell lysates, may be determined by known assays, such as p-Nitrophenyl Phosphate (PNPP) assay. Collagen levels, for example in cell lysates, may also be determined using known assays, such as the Sircoll dye-based assay. The lectin may be capable of increasing the level of ALP activity by 5-50% or 10-40%, relative to a suitable control which has not been treated with the lectin. The lectin may be capable of increasing the level of collagen by 2-50% or 3-40%, relative to a suitable control which has not been treated with the lectin.

As used herein, the term "inflammation" relates to any type of inflammatory immune response in a subject. The inflammation may be associated with a chronic inflammatory disease, or it may be acute inflammation.

Chronic inflammation is long-term inflammation and can last for several months and even years, whereas acute inflammation is one that starts rapidly and becomes severe in a short span of time. Chronic inflammations include Psoriasis/psoriatic arthritis, Rheumatoid arthritis (RA), active hepatitis, asthma, and inflammatory bowel disease, which need to be well managed and treated well in time. The damages caused by chronic inflammations are irreversible and highly painful.

The terms "chronic inflammations" and "chronic inflammatory diseases" are used herein interchangeably throughout.

In some embodiments the subject has a chronic inflammatory disease. Advantageously, the lectin protein can be used in the treatment or prevention of a chronic inflammatory disease in the subject.

The chronic inflammatory disease may be psoriasis, psoriatic arthritis, rheumatoid arthritis (RA), hepatitis, asthma, inflammatory bowel disease (such as Crohn's disease, colitis or irritable bowel syndrome (IBS)), temporal arteritis, atopic dermatitis, systemic lupus, multiple sclerosis or sarcoidosis.

In some embodiments, the chronic inflammatory disease is rheumatoid arthritis (RA).

Rheumatoid Arthritis can be measured or diagnosed using the following factors.

Antinuclear antibody (ANA) --- Commonly found in the blood of people who have lupus, ANAs (abnormal antibodies directed against the cells' nuclei) can also suggest the presence of polymyositis, scleroderma, Sjogren's syndrome, mixed connective tissue disease or rheumatoid arthritis. Tests to detect specific subsets of these antibodies can be used to confirm the diagnosis of a particular disease or form of arthritis.

In some embodiments, a subject is regarded as having rheumatoid arthritis if the subject has detectable levels of antinuclear antibody.

Rheumatoid factor (RF) --- Designed to detect and measure the level of an antibody that acts against the blood component gamma globulin, this test is often positive m people with rheumatoid arthritis. Levels of 20 IU/mL or higher are considered indicative of disease.

In some embodiments, a subject is regarded as having rheumatoid arthritis if the subject has a rheumatoid factor level of at least 20 IU/mL.

Anti-cyclic citrullinated peptide (anti-CCP) - Also called anti-citrullinated protein antibodies (ACPA), this test (like the test for rheumatoid factor) looks for the presence of a particular autoantibody that is present in approximately 60-80 percent of people with RA. While most patients with anti-CCP antibodies are also positive for rheumatoid factor, the RF antibody can occur in patients with many other conditions, including an infection. Anti-CCP is more specific for RA. and is becoming the preferred test.

In some embodiments, a subject is regarded as having rheumatoid arthritis if the subject has higher than 20 units per milliliter (u/ml) detectable levels of anti- citrullinated protein antibodies.

Uric acid --- By measuring the level of uric acid m the blood, this test helps diagnosis of gout, a condition that occurs when excess uric acid crystallizes and forms deposits m the Joints and other tissues, causing inflammation and severe pain. Normal uric acid levels are 2.4-6.0 mg/dL in females and 3.4-7.0 mg/dL in males.

In some embodiments, a subject is regarded as having rheumatoid arthritis if the subject has uric acid levels of at least 6.0 mg/dL, or at least 7.0 mg/dL

HLA tissue typing - This test, which detects the presence of certain genetic markers m the blood, can often confirm a diagnosis of ankylosing spondylitis (a disease involving inflammation of the spine and sacroiliac joint) or reactive arthritis (a disease involving inflammation of the urethra, eyes and joints). The genetic marker HLA-B27 is almost always present in people with either of these diseases.

In some embodiments, a subject is regarded as having rheumatoid arthritis if the subject has the genetic marker HLA-B27.

Erythrocyte sedimentation rate --- Also called ESR or "sed rate," this test measures how fast red blood cells cling together, fall and settle (like sediment) in the bottom of a glass tube over the course of an hour. The higher the sed rate, the greater the amount of inflammation. This sed rate is measured in the distance taken for the erythrocytes to fall down the tube in an hour. The normal range 1s 0-29mm/hr for females and 0-20 mm/hr for males. There are many conditions that can cause an elevated ESR, including an infection or anemia.

In some embodiments, a subject is regarded as having rheumatoid arthritis if the subject has an erythrocyte sedimentation rate of at least 20 mm/hr, or at least 29 mm/hr.

C-reactive protein --- This test, also called CRP, is another blood test that measures body-wide inflammation. It measures a substance produced by the liver that increases m the presence of inflammation.

In some embodiments, a subject is regarded as having rheumatoid arthritis if the subject has blood with a concentration of C-reactive protein of at least 1.0 mg/L, at least 3.0 mg/L, or at least 10.0 mg/L.

Lyme serology --- This test detects an immune response to the infectious agent that causes Lyme disease and thus can be used to confirm a diagnosis of the disease.

In some embodiments, the subject has acute inflammation. Advantageously, the lectin protein can be used in the treatment or prevention of acute inflammation in a subject.

Acute inflammation may be caused by an allergic reaction, exposure of the subjection to a chemical or radiation, an infection or trauma. The infection may be bacterial, viral, fungal or parasitic.

It will be understood that the term "treatment" comprises substantially curing (i.e. eliminating) the inflammation or an underlying chronic inflammatory disease, or reducing (either permanently or temporarily) the symptoms associated with the inflammation. Such symptoms may include swelling, pain, itching, heat, redness (e.g. of the skin), loss of function (e.g. of joints or limbs), and difficulty breathing.

It will be understood that the term "prevention" may comprise preventing the onset of the inflammation, or preventing or slowing the progression of the inflammation or of an underlying chronic inflammatory disease.

The treatment or prevention may comprise administering a therapeutically effective amount of the lectin to the subject. In some embodiments, the lectin is administered at a dose of from 0.1 to 1000 mg/kg, from 0.5 to 100 mg/kg or from 1 to 50 mg/kg. It will be within the capabilities of the skilled person to determine an amount of lectin to be administered according to the nature of the condition being treated and the subject.

The present invention thus also provides a pharmaceutical composition comprising a lectin protein and a pharmaceutically acceptable diluent or excipient., wherein the lectin protein is recombinant, and is derived from *Sclerotium rolfsii,* and comprises an amino acid sequence of SEQ ID NO:2 for use in a method of treatment or prevention of inflammation in a subject, the method comprising administering the pharmaceutical composition to the subject. Exemplary diluents and excipients include sterilised water, physiological saline, and/or pharmaceutically acceptable buffer.

Administration of the lectin or composition may be by any suitable route, including but not limited to, injection (including intravenous (bolus or infusion), intra-arterial, intraperitoneal, subcutaneous (bolus or infusion), intraventricular, intramuscular, or subarachnoidal), oral ingestion (e.g. of a tablet, gel, lozenge or liquid), inhalation, topical, via a mucosa (such as the oral, nasal or rectal mucosa), by delivery in the form of a spray, tablet, transdermal patch, subcutaneous implant or in the form of a suppository.

In some embodiments, a lectin or a pharmaceutical composition as described herein is administered to the subject enterally, parenterally or topically. The lectin or pharmaceutical composition may be administered as a dosage form which is solid (such as tablet or capsule), a lyophilized powder, a liquid (such as solution or suspension), a semi-solid or any other form as known to the person skilled in the art. The lectin or the pharmaceutical composition may be administered to the subject by injecting a solution or suspension intravenously, intramuscularly, intraperitoneally, subcutaneously, or intradermally, by depot injection, or it may be administered intrathecally, transdermally, sublingually or by oral, topical or inhalation methods.

The subject may be a mammalian subject. In some embodiments, the subject is human. In particular, the subject may be a human subject suffering from or seeking prevention from inflammation or inflammatory disease.

**Description of Drawings and Tables**

The invention will now be described by reference to the following Figures and Tables, in which:

Fig I: Effect of a recombinant lectin on the inhibition of inflammatory cytokines in an *in vivo* study using an LPS-induced cytokine release mouse model;
Fig 2: Effect of a recombinant lectin on paw swelling using an Adjuvant Induced Arthritis (AIA) rat model;
Fig 3: Effect of a recombinant lectin on bone destruction using an Adjuvant Induced Arthritis (AIA) rat model;
Fig 4: Effect of a recombinant lectin on inhibition of the CRP level and IL-6 using an Adjuvant Induced Arthritis (AIA) rat model;
Table 1: Effect of a recombinant lectin on cytokine secretion in a human synovial cell line (SW982) after 24 hours;
Table 2. Effect of a recombinant lectin on cytokine secretion in murine splenocytes after 48 hours;
Table 3. Effect of SEQ ID NO:2 on cytokine secretion in a murine macrophage cell line (RAW264.7) after 24 hours; and
Table 4. Effect of SEQ ID NO:2 on ALP activity and collagen levels in an osteoblast cell line (MG-63) after 5 days.

Inflammatory diseases are diseases caused by the impairment of immune system. Inflammatory diseases can be chronic inflammatory disease or acute inflammatory disease. Inflammatory diseases include arthritis, psoriasis, temporal arteritis, dermatitis, inflammatory bowel disease (IBS), systemic lupus, hepatitis, asthma, allergic reactions, sarcoidosis and similar diseases as understood by the person skilled m the art.

Key signaling pathways which play an important role in the pathogenesis of inflammation are MAPK/EGFR/-Ras/Raf, Cannabinoid receptor 2 (CNR2), IL- 4/STAT, PI3K/AKT/FOXO3, TNF-$\alpha$/JNK, NLRP3 and NLRPI, *PTPN22, PADI4, STAT4, TRAFJ-C5* and *TNFAIP3,* MyD88, IRAKs, and TRAF6.

The present disclosure relates to the treatment of inflammatory diseases using a lectin according to SEQ ID NO:2. The disclosure also relates to the use of the lectin in a method for the prevention of, the onset of or the progression of inflammatory diseases in the subject. It further relates to the use of the lectin in a method to cure or reduce the effect of inflammatory diseases in the subject.

The present disclosure relates to the use of a lectin according to SEQ ID NO: 2 in a method of treatment of arthritis in a subject.

The invention also provides a lectin of SEQ ID NO: 2 for use in a method for the prevention of onset of or progression of arthritis in the subject. Further the invention relates to a lectin of SEQ ID NO: 2 for use in a method to cure or reduce the effect of arthritis in a subject using a recombinant lectin having the amino acid sequence of SEQ ID NO: 1 or its homologues.

The applicant's earlier patent application, 350/MUM/2009, discloses a recombinant lectin with the amino acid sequence of SEQ ID NO: 1 having high binding specificity toward the carbohydrate antigen (Galß1-3GalNAc-a-O-Ser/Thr)). Lectins are generally known to cause inflammation. However, the present inventors, to their surprise, have found that the lectin with the amino acid sequence of SEQ ID NO: 1, 2, 3, or 4 is anti-inflammatory. The detailed study confirmed the efficacy of recombinant lectin with the amino acid sequence of SEQ ID NO: 2 towards arthritis. The novel and inventive aspect of the present invention is the use of a recombinant lectin with the amino acid sequence of SEQ ID NO: 2 in the prevention, cure or reduction of proliferation of inflammation, such as that causing arthritis.

The effect of a recombinant lectin having the sequence of SEQ ID NO:2 on the modulation of key signaling pathways which play an important role in the pathogenesis of inflammation was evaluated in cell-based assays. A recombinant lectin having the amino acid sequence of SEQ ID NO: 2 showed inhibition of signaling pathways which are key pathways in the pathogenesis of inflammation related to arthritis. These results, detailed below, suggest the possible efficacious effect of lectins, such as the recombinant lectin having the amino acid sequence of SEQ ID NO: 2 in inflammation related to arthritis.

*In vitro* studies of a recombinant lectin having the amino acid sequence of SEQ ID NO: 2 demonstrated strong anti-inflammatory activity by inhibiting pro-inflammatory cytokines and inhibition of signaling pathways which play a key role in the pathogenesis of inflammation. It also stimulated ALP activity and collagen levels in human osteoblast cells supporting its anti-inflammatory potential.

Synovial fibroblasts produce excessive levels of inflammatory cytokines which destroy cartilage and bone in inflamed arthritic joint. A human synoviocyte cell line is characterized by expression of inflammatory cytokines. *In-vitro* cell based assays on human synoviocyte cell lines were performed using a recombinant lectin having the amino acid sequence of SEQ ID NO: 2 to assess its effect on the secretion of inflammatory cytokines such as interleukins, interferons, granulocyte-macrophage colony-stimulating factor, (GM-CSF), PI3K8/y and TNF-$\alpha$ against IFN-$\gamma$ stimulation. The effect of using a recombinant lectin having the amino acid SEQ ID NO:2 in an *in-vitro* cell based LPS induction assay on the immune cells murine splenocytes and an *in-vitro* cell-based IFN-$\gamma$ stimulation assay on the murine macrophage cell line RAW264.7 were also assessed. A recombinant lectin having the amino acid sequence of SEQ ID NO: 2 significantly inhibited the secretion of inflammatory cytokines. The secretion of IL-8 and IL-6 was inhibited by 10% to 30% and 20% to 50%, respectively, in synovial cells against interferon gamma (IFN-$\gamma$) stimulation (Table 1). Similarly, a recombinant lectin having the amino acid sequence of SEQ ID NO: 2 inhibited the secretion of TNF-$\alpha$ and IL-1-$\beta$ by 15 to 35 % and 20 to 32% respectively in splenocytes stimulated with LPS (Table 2). The inhibition of the secretion of TNF-$\alpha$ in a murine macrophage (RAW264.7) cell line stimulated with IFN-$\gamma$ was from 5 to 30% (Table 3). The results demonstrated that a recombinant lectin having the amino acid sequence of SEQ ID NO: 2 inhibits key inflammatory cytokines in a synovial cell line as well as immune cells.

A recombinant lectin having the amino acid sequence of SEQ ID NO: 2 was assessed for its effect on the stimulation of bone markers such as Alkaline Phosphatase (ALP) activity and collagen levels in a human osteoblast cell line. A recombinant lectin having the amino acid sequence of SEQ ID NO: 2 stimulated ALP activity in osteoblasts by 10% to 45% with respect to the control. A recombinant lectin having the amino acid sequence of SEQ ID NO. 2 also stimulated collagen levels by 3% to 50% as compared to the control (Table 4).

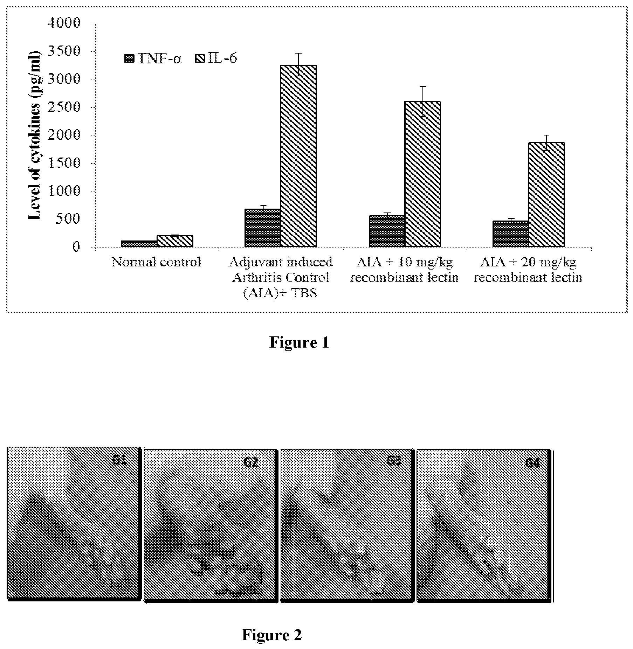

A recombinant lectin having the amino acid sequence of SEQ ID NO: 2 was also tested for its effect on the inhibition of inflammatory cytokines in an *in vivo* study using an LPS-induced cytokine release mouse model. The mice were treated with lipopolysaccharide (LPS) alone (50 $\mu$g/animal) and LPS (50 $\mu$g/animal) along with a recombinant lectin having the amino acid sequence of SEQ ID NO: 2 (5 to 50 mg/kg body weight). The dosing of recombinant lectin having the sequence of SEQ ID NO: 2 was done 1 hour before the injection of LPS. Normal control animals received TBS (tris-buffered saline), at the dose volume of 5 mL/kg. All doses were administered intraperitoneally. Excessive secretion of TNF-$\alpha$ (675.7 $\pm$ 76.6 pg/mL) and IL-6 (3256.0 $\pm$ 203.1 pg/mL) was observed in the blood serum when LPS alone was injected. The recombinant lectin having the amino acid sequence of SEQ ID NO: 2 significantly inhibited the secretion of cytokines (Fig 1). In particular, the recombinant lectin having the amino acid sequence SEQ ID NO: 2 inhibited the secretion of TNF-$\alpha$ by up to 30% (471.8 $\pm$ 48.3 pg/mL) at a concentration of about 20 mg/kg body weight respectively. Similarly, the secretion of IL-6 was inhibited up to 43% (1866.2 $\pm$ 139.3 pg/mL) at concentration of about 20 mg/kg body weight.

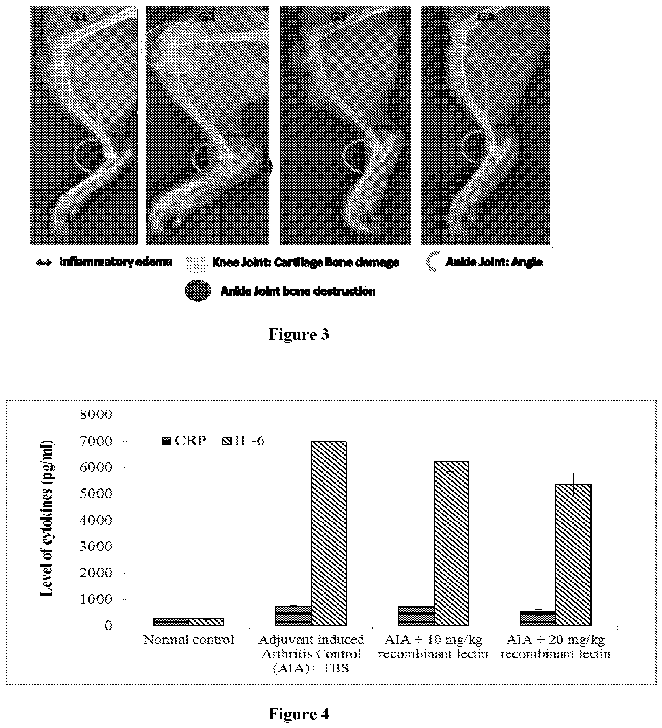

The effect of the recombinant lectin having the sequence of SEQ ID NO: 2 was further tested on an adjuvant induced arthritis (AIA) model in rats. Animals were injected with a recombinant lectin having the amino acid sequence of SEQ ID NO: 2 intraperitoneally at a concentration of 20 mg/kg of body weight for 21 days (from day 8 to 28 after adjuvant injection). Treatment with the recombinant lectin having the amino acid sequence of SEQ ID NO: 2 significantly reduced paw and ankle thickness compared to vehicle-treated adjuvant-induced arthritis animals (Fig 2). Scoring of ankle joint radiography from the vehicle-treated adjuvant-induced arthritic animals showed the severe destruction of bone when compared to normal control animals. Significant amelioration of bone destruction was evident in animals treated with the recombinant lectin having the amino acid sequence of SEQ ID NO: 2(Fig 3). The vehicle-treated adjuvant-induced arthritis group showed a significant decrease in joint angle when compared to the normal control. Animals treated with about 20 mg/kg of a recombinant lectin having the amino acid sequence of SEQ ID NO. 2, dose dependently, increased the joint mobility with the percentage improvement up to 34.2%, respectively. The effect was shown to be dose dependent as the lower doses of recombinant lectin showed lower improvement in joint mobility. Further, treatment with the recombinant lectin showed reduced levels of serum uric acid compared to vehicle-treated adjuvant-induced arthritic animals, up to 22%. C Reactive Protein (CRP) is produced by the liver and can be found in the blood. Generally, like cytokines, CRP levels in blood also rise in response to inflammation. Vehicle-treated adjuvant-induced arthritic animals showed increased serum CRP and IL-6 levels. A significant decrease in the CRP levels was observed in animals treated with the recombinant lectin having the amino acid sequence of SEQ ID NO: 2(Fig 4). The present description demonstrates that the recombinant lectin having the amino acid sequence of SEQ ID NO:2 significantly reduces the progression and severity of arthritis in a dose-dependent manner in the FCA (Freund's complete adjuvant)- induced arthritis rat model. The present invention thus provides a recombinant lectin having the amino acid sequence of SEQ ID NO:2 for use in a method of treatment of inflammatory diseases. It can be used in the method of treatment, prevention of onset of or progression of or to cure, to reduce the effect of inflammatory diseases in the subject.

The method may comprise administration of a lectin having the amino acid sequence of SEQ ID NO: 1 or its homologues in the range of about 1 mg to 50 mg/kg body weight of the subject, wherein the recombinant lectin having the amino acid sequence of SEQ ID NO: 1 is administered as such or in the pharmaceutically acceptable form.

A preferred range of the recombinant lectin is from about 5 mg to 30 mg/kg body weight of the subject. A most preferred range of the recombinant lectin is from about 5 mg to 20 mg/kg body weight of the subject.

An anti-arthritic effect of a recombinant lectin having an amino acid sequence of SEQ ID 2 is at least 10% to up to 100%.

## Examples

The following examples are given to demonstrate the best mode of performance of the invention. Examples do not limit the invention in any manner.

**Example 1:**

A study was conducted to assess the effect of a recombinant lectin having the amino acid sequence of SEQ ID NO:2 on the secretion of inflammatory cytokines (IL-8 and IL-6) in a human synoviocyte cell line (SW982). The recombinant lectin showed inhibition of the secretion of the inflammatory cytokines from the cell line.

Human synoviocyte (SW982) cells were first cultured for 24 hours and sera starved for 6 hours, followed by treatment of the cells with the recombinant lectin having the amino acid sequence of SEQ ID NO: 2at non-cytotoxic concentrations (1 ng/ml - 1000 ng/ml) with the inflammatory stimulus (100ng/ml IFN-$\gamma$) for another 24 hours. The levels of cytokines (IL-6 and IL-8) in supernatants were determined by ELISA. Inhibitory effect of recombinant lectin on secretion of cytokine was calculated as follows:

$$\% \text{ inhibition of cytokines} = [(A-B)/A]*100$$

Where,

A = concentration of cytokine in control cells (IFN-$\gamma$ alone)
B = concentration of cytokine in treated cells (recombinant lectin + IFN-$\gamma$ )

It was seen that the recombinant lectin having the amino acid sequence of SEQ ID NO: 2inhibited IL-8 secretion from synovial cells (SW982) against IFN-$\gamma$ stimulation (11.2% - 27.9%) and inhibited IL-6 secretion from synovial cells (SW982) against IFN-$\gamma$ stimulation (23.7% - 49.4%).

**Example 2:**

Another study was conducted to determine the effect of a recombinant lectin having the amino acid sequence of SEQ ID NO: 2 on the secretion of inflammatory cytokines (TNF-α and IL-1-β) from immune cells (murine splenocytes and the munne macrophage cell line (RAW264.7)).

Splenocytes were isolated from C57BL/6 mice and treated with a recombinant lectin having the amino acid sequence of SEQ ID NO: 2at non-cytotoxic concentrations (1 ng/ml - 1000 ng/ml) along with 25ng/ml of LPS for 48 hours.

RAW264.7 cells were cultured for 24 hours and sera starved for another 24 hours followed by co-treatment of the cells with a recombinant lectin having the amino acid sequence of SEQ ID NO: 2at non-cytotoxic concentrations (Ing/ml - 500 ng/ml) and the inflammatory stimulus IFN-γ (100 ng/ml) for 24 hours. Levels of cytokines (TNF-α and IL-1-β) in supernatants were determined by ELISA

Inhibitory effect of recombinant lectin on secretion of cytokine was calculated as follows:

$$\% \text{ inhibition of cytokines} = [(A-B)/A]*100$$

Where,

A= concentration of cytokine in control cells (LPS or IFN-γ alone)
B = concentration of cytokine in treated cells (recombinant lectin + LPS or IFN- y)

It was observed that in murine splenocytes, the recombinant lectin having the amino acid sequence of SEQ ID NO: 2 demonstrated the inhibition of the secretion of cytokines from splenocytes against LPS stimulation (TNF-α by 17.2% - 32.9% and IL-1-β by 23.5% - 30.4%).

In murine macrophages (RAW264.7), the recombinant lectin having the amino acid sequence of SEQ ID NO: 2inhibited TNF-α secretion from macrophages (RAW264.7) against LPS stimulation (6.1%-26.5%)

**Example 3:**

Another study was conducted to determine the effect of a recombinant lectin having the amino acid sequence of SEQ ID NO: 2 on the bone markers (ALP & Collagen) in a human osteoblasts cell line (MG-63). First, MG-63 cells were cultured for 24 hours and sera starved for another 24 hours followed by treatment of the cells with a recombinant lectin having the amino acid sequence of SEQ ID NO: 2at non-cytotoxic concentrations (Ing/ml - 1000 ng/ml) for 5 days. ALP activity in cell lysates was determined by a PnPP based assay. Collagen levels in cell lysates were determined by a Sircoll dye-based assay. The recombinant lectin having the amino acid sequence of SEQ ID NO: 2 increased ALP activity (11% - 42.8%) and collagen levels (3.2% - 45.3%) in the human osteoblasts cell line (MG-63).

**Example 4:**

Another study was conducted to consider the effect of a recombinant lectin having the amino acid sequence of SEQ ID NO: 2on key signaling pathways which play an important role in the pathogenesis of Rheumatoid Arthritis.

The effect of a recombinant lectin having the amino acid sequence of SEQ ID NO: 2on the modulation of key signaling pathways was determined in cell-based assays using Geneblazer and Tango technologies.

It was seen that the recombinant lectin having the amino acid sequence of SEQ ID NO: 2 inhibited the following pathways: MAPK/EGFR/Ras/Raf, Cannabinoid receptor 2 (CNR2), IL-4/STAT, PI3K/AKT/FOXO3 and TNF-α/JNK, further strengthening the suggested role of the lectin having the amino acid sequence of SEQ ID NO: 1 or its homologues in its favor in the case of rheumatoid arthritis.

***In vivo* Experiments**

**Example 5:**

Another study was conducted to check the effect of a recombinant lectin having the amino acid sequence of SEQ ID NO: 2 on inflammatory cytokines (TNF-α and IL6) in an LPS-induced cytokine release model in mice.

30 female C56BL6 mice were randomized based on body weight and assigned to five groups (n=6). For groups receiving LPS (G2), a single dose of 50μg/animal of LPS was injected intraperitoneally (IP). For groups receiving a recombinant lectin having the amino acid sequence of SEQ ID NO: 2 at 10 & 20mg/kg +LPS, (G3 & G4) a single IP injection of 10 & 20mg/kg of the recombinant lectin having the amino acid sequence of SEQ ID NO: 2 was administered 1hr before the injection of LPS. For a group of Dexamethasone (G5), Img/kg +LPS, a single IP injection of Img/kg of dexamethasone was administered 1hr min before injection of the LPS. Normal control animals (GI) received TBS intraperitoneally at the dose volume of 5 mL/kg.

Blood was collected 90 min after treatment with LPS and the blood was centrifuged for the collection of serum. The serum samples were stored at -80 °C. Serum TNF-$\alpha$ and IL-6 were measured using an ELISA kit.

**Serum cytokine levels**

TNF-$\alpha$ was 102.3 $\pm$ 9.2 pg/mL in the serum of TBS treated mice, while the IL-6 concentration in the serum of TBS-treated mice was 204.0 $\pm$ 14.3 pg/mL.

A significant increase in the serum level of TNF-$\alpha$ was observed in the LPS group at 90 minutes to an average of 675.7 $\pm$ 76.6 pg/mL. This was inhibited by the recombinant lectin having the amino acid sequence of SEQ ID NO: 2to 566.2 $\pm$ 52.3 and 471.8 $\pm$ 48.3 pg/mL for 10mg/kg and 20mg/kg respectively, giving a percentage reduction of 16.2% and 30.2%, respectively.

A significant increase in the serum level of IL-6 was observed in the LPS group at 90 minutes to an average of 3256.0 $\pm$ 203.1 pg/mL. This was inhibited by the recombinant lectin having the amino acid sequence of SEQ ID NO: 2to 2601.3 $\pm$ 268.2 and 1866.2 $\pm$ 139.3 pg/mL for 10mg/kg and 20mg/kg, respectively, giving a percentage reduction of 20.1% and 42.7%, respectively.

In short, it is demonstrated that the lectin having the amino acid sequence of SEQ ID NO: 1 or its homologues has a promising anti-inflammatory activity due to its ability to suppress the pro-inflammatory cytokines, TNF-$\alpha$ and IL-6 in an LPS-induced mouse inflammation model.

**Example 6:**

Another study was conducted to see the effect of a recombinant lectin having the amino acid sequence of SEQ ID NO:2 on an adjuvant induced-arthritis model in rats.

Adjuvant arthritis was induced by the injection of 100$\mu$L (0.5 mg) of *Mycobacterium butyricum* desiccated - Complete Freund's Adjuvant-CFA (Chondrex) into the left hind paw (Day 0) of 34 rats. On day 0 of the experiment, 24 animals were selected, randomized and assigned to four groups of 6 animals each.

The groups were as follows:

**G1:** Normal Control
**G2:** Adjuvant induced arthritis control (AIA) + TBS
**G3:** AIA + IO mg/Kg recombinant lectin
**G4:** AIA + 20 mg/Kg recombinant lectin
**G5:** AIA + 0.5mg/kg dexamethasone

A recombinant lectin having the amino acid sequence of SEQ ID NO:2 compound was administered from days 8 to 28 to two groups (G3 and G4) after adjuvant injection, a total of 21 days treatment. Control groups were administered the following. Normal control animals (GI, n=6) were injected with 100$\mu$L of paraffin oil into the left hind paw on day 0 and then dosed on days 8 to 28 with Tris Buffer Saline, 5mL/kg, IP. Another control group received Complete Freund's Adjuvant (CFA) on day 0 followed by the intraperitoneal injection of Tris Buffer Saline on days 8 to 28 (G2). For the groups receiving the recombinant lectin having the amino acid sequence of SEQ ID NO:2, IP injection of 10 & 20 mg/kg of the drug (G3 & G4 respectively), was administered daily for 21 days (days 8 to 28). For the group of Dexamethasone (G5), an IP injection of 0.5mg/kg of dexamethasone was administered daily for 21 days (days 8 to 28) following CFA on day 0.

Paw thickness was measured by a digimatic micrometer (Mitutoyo). The angle of the ankle joint by degree was also recorded. On day 28 after adjuvant injection and day 21 post treatment, whole blood was collected from the animals and centrifuged to separate serum. The serum samples were stored at -80°C. Serum Uric acid, C-Reactive Protein (CRP) and IL-6 was measured using kits mentioned below.

| Name of Kit | Manufacturer | Catalogue no. |
|---|---|---|
| Rat C-Reactive Protein,CRP ELISA Kit | CUSABIO | CSB-E07922r |
| Rat Interleukin 6,IL-6 ELISA KIT | CUSABIO | CSB-E04640r |
| Rat Uric Acid (UA) ELISA Kit | MYBIOSOURCE | MBS9346935 |

Assessment of joint destruction (Radiographic evaluation):

On the day of termination (day 28), the hind limb was sectioned below the hip joint. Radiographs were taken of the hind limb. The severity of bone damage on the radiographs was assessed by the grading of periostitis and bone destruction.

Radiography of the hind limb from the vehicle-treated adjuvant-induced arthritis group (G2), showed severe disorganization (periostitis and destruction) of bone in the distal tibia, tarsus, metatarsus, and calcaneus. 21 days of intraperitoneal injection of 20mg/kg a recombinant lectin having the amino acid sequence of SEQ ID NO: 2, moderately ameliorated this periostitis and bone destruction (G4) whereas 10 mg/kg of a recombinant lectin having the amino acid sequence of SEQ ID NO: 2showed a palpable effect (G3). Dexamethasone 0.5 mg/kg, IP showed a significant amelioration (G5).

**Effect of a recombinant lectin having the amino acid sequence of SEQ ID NO: 2on ankle bone radiography in adjuvant-induced rat model of arthritis**

Ankle joint radiography from the vehicle-treated adjuvant-induced arthritis group (G2) showed the severe destruction of bone when compared to normal animals (GI). 21 days of an IP injection of 10 mg/kg and 20 mg/kg (G3 and G4) of a recombinant lectin having the ammo acid sequence of SEQ ID NO: 2, mild to moderately ameliorated this bone destruction. Dexamethasone 0.5mg/kg, IP showed significant amelioration (G5).

**Effect of a recombinant lectin having the amino acid sequence of SEQ ID NO: 2on joint angle (Joint Mobility) in an adjuvant-induced rat model of arthritis**

The vehicle-treated adjuvant-induced arthritis group (G2) showed a significant decrease in joint angle when compare to the normal control group (G1). 21 days of an intraperitoneal injection of 10 mg/kg and 20 mg/kg of a recombinant lectin having the amino acid sequence of SEQ ID NO: 2(G3 & G4), dose dependently increased the joint mobility with a percentage improvement of 7.9 % and 34.2%, respectively. Dexamethasone 0.5mg/kg, showed significant amelioration (G5) with 61.8%.

**Inhibitory effects of a recombinant lectin having the amino acid sequence of SEQ ID NO: 2on paw and ankle thickness in an adjuvant-induced rat model of arthritis**

The vehicle-treated adjuvant-induced arthritis group (G2) showed a significant increase in paw and ankle thickness. 21 days of intraperitoneal injection of 20mg/kg of a recombinant lectin having the amino acid sequence of SEQ ID NO: 2, significantly reduced paw and ankle swelling (G4), whereas 10 mg/kg showed a poor effect (G3). Dexamethasone 0.5mg/kg, IP, showed significant amelioration (G5).

**Effect of a recombinant lectin having the amino acid sequence of SEQ ID NO: 2on serum uric acid levels in an adjuvant-induced rat model of arthritis**

Dexamethasone decreased the level of serum uric acid significantly (G5) in adjuvant-induced rats with arthritis. 21 days of intraperitoneal injection of a recombinant lectin having the amino acid sequence of SEQ ID NO: 2 at 10mg/kg and 20mg/kg dose (G3 and G4) dependently decreased the uric acid level with a percentage reduction of 11.0% and 22.0%, respectively.

**Effect of a recombinant lectin having the amino acid sequence of SEQ ID NO: 2on serum IL-6 and CRP levels in an adjuvant-induced rat model of arthritis**

C-Reactive Protein (CRP) was $290.6 \pm 15.5$ pg/mL in the serum of Tris Buffered Saline (TBS) treated rats (GI), while the IL-6 concentration in the serum of TBS treated rats was $265.8 \pm 22.7$ pg/mL. A significant increase in the serum level ofIL-6 was observed in the arthritis group (G2) to an average of $6989.2 \pm 475.2$ pg/mL. This was inhibited by a recombinant lectin having the amino acid sequence of SEQ ID NO: 2 to 6219.8 $\pm$ 359.8 and 5393.6 $\pm$ 420.4 pg/mL for 10mg/kg and 20mg/kg dosages respectively (G3 and G4), giving a percentage reduction of 11.0% and 22.8%, respectively.

A significant increase in the serum level of CRP was observed in the Arthritis group (G2) to an average of 748.2 $\pm$ 21.8 pg/mL. This was inhibited by a recombinant lectin having the amino acid sequence of SEQ ID NO: 2to 726.4 $\pm$ 36.6 and 523.3 $\pm$ 110.5 pg/mL for 10mg/kg and 20mg/kg (G3 and G4), respectively, giving a percentage reduction of 2.9% and 30.1%, respectively.

**Effect of a recombinant lectin having the amino acid sequence of SEQ ID NO: 2and dexamethasone on thymus and spleen weight in an adjuvant-induced rat model of arthritis**

The vehicle-treated adjuvant-induced arthritis group (G2) showed a significant decrease in thymus weight when compared to the normal control group (GI). 21 days of intraperitoneal injection of 10mg/kg and 20mg/kg of a recombinant lectin having the amino acid sequence of SEQ ID NO: 2 (G3 & G4), showed 14.48% gain and 3.98% loss, respectively compared to the G2 group. Dexamethasone 0.5mg/kg, IP, showed a considerable reduction (i.e. 19.33%) in thymus weight compared to the G2 group.

The vehicle-treated adjuvant-induced arthritis group (G2) showed a marginal increase in spleen weight when compared to the normal control group (GI). 21 days of intraperitoneal injection of 10mg/kg and 20mg/kg of a recombinant lectin having the amino acid sequence of SEQ ID NO: 2 (G3 & G4), showed a 40.55% and 12.17% increase, respectively compared to the G2 group. Dexamethasone 0.5mg/kg, IP, showed a significant reduction (i.e. 51.57%) in spleen weight compared to the G2 group.

**Effect of a recombinant lectin having the amino acid sequence of SEQ ID NO: 2and dexamethasone on hematology in an adjuvant-induced rat model of arthritis**

The vehicle-treated adjuvant-induced arthritis group (G2) showed a moderate to significant alteration in all the tested hematological parameters when compared to the normal control group (GI). 21 days of intraperitoneal injection of 10mg/kg and 20mg/kg of a recombinant lectin having the amino acid sequence of SEQ ID NO: 2 (G3 & G4), did not alter the change caused by arthritis. However, Dexamethasone 0.5mg/kg, IP, showed moderate to significant changes (i.e. WBC-60.3% reduction, RBC - 23.4% Increase, Hb-25.2% increase and HCT-22.4% increase) in hematology compared to the G2 group.

**Effect of a recombinant lectin having the amino acid sequence of SEQ ID NO: 2and dexamethasone on body weight in an adjuvant-induced rat model of arthritis**

Recombinant lectin treated groups (G3 & G4) and the vehicle-treated adjuvant- induced arthritis group (G2), showed a similar trend to the normal control group (GI) in gaining body weight. However, the dexamethasone 0.5mg/kg, IP group (G5) showed no gain in body weight throughout the treatment period.

Table 1.

| Sample | Inhibition of cytokines (%) | |
|---|---|---|
| | IL-8 | IL-6 |
| IFN-$\gamma$ 100 ng/ml | Control | Control |
| IFN-$\gamma$ 100 ng/ml + SEQ ID NO:2 (ng/ml) | | |
| I | 22.4 | 46.3 |
| 10 | 27.9 | 49.4 |
| 250 | 14.9 | 31.9 |
| 500 | 11.2 | 23.7 |
| 1000 | -40.8 | -25.2 |

Table 2.

| Sample | Inhibition of cytokines (%) | |
|---|---|---|
| | *TNF-$\alpha$* | IL-1-1} |
| LPS 25 ng/ml | Control | Control |
| LPS 25 ng/ml + SEQ ID NO:2(ng/ml) | | |
| 1 | 24.8 | 30.4 |
| 10 | 18.6 | 24.4 |
| 25 | 29.2 | 23.5 |

(continued)

| Sample | Inhibition of cytokines (%) | |
|---|---|---|
| | *TNF-α* | IL-1-1} |
| LPS 25 ng/ml | Control | Control |
| LPS 25 ng/ml + SEQ ID NO:2(ng/ml) | | |
| 100 | 32.9 | 27.6 |
| 500 | 26.1 | 26.7 |
| 1000 | 17.2 | 24.4 |

**Table 3.**

| Sample | Inhibition of *TNF-α* (%) | |
|---|---|---|
| IFN-γ 100 ng/ml | Control | |
| IFN-γ 100 ng/ml + SEQ ID NO:2(ng/ml) | | |
| 1 | 26.5 | |
| 10 | 6.1 | |
| 100 | 11.7 | |
| 500 | 11 | |

**Table 4.**

| Sample | % increase in ALP activity wrt to control | % increase in collagen level |
|---|---|---|
| Recombinant lectin | | |
| 1 | 37.7 | 3.2 |
| 10 | 42.8 | 45.3 |
| 25 | 30.9 | 26.7 |
| 100 | 27.3 | 26.8 |
| 1000 | 11 | 13.5 |
| Vitamin D3 (Positive control) | | |
| 1 | 31.6 | 14 |
| 10 | 60.8 | 30.9 |

## Claims

1. A lectin protein for use in the treatment or prevention of inflammation in a subject in need thereof, wherein the lectin protein is recombinant, is derived from *Sclerotium rolfsii,* and comprises an amino acid sequence of SEQ ID NO:2.

2. The lectin protein for use as claimed in any preceding claim, wherein the treatment or prevention of inflammation comprises a) inhibiting the secretion of inflammatory cytokines; and/or b) reducing C-reactive protein (CRP) levels in blood; and/or c) stimulating ALP activity and/or collagen levels.

3. The lectin protein for use as claimed in any preceding claim, wherein the subject has a chronic inflammatory disease.

4. The lectin protein for use as claimed in claim 3, wherein the chronic inflammatory disease is psoriasis, psoriatic arthritis, rheumatoid arthritis (RA), hepatitis, asthma, inflammatory bowel disease (including crohn's disease, colitis and irritable bowel syndrome (IBS)), temporal arteritis, atopic dermatitis, systemic lupus, multiple sclerosis or sarcoidosis.

5. The lectin protein for use as claimed in claim 4, wherein the chronic inflammatory disease is rheumatoid arthritis (RA).

6. The lectin protein for use as claimed in one of claims 1 to 2 , wherein the subject has acute inflammation.

7. The lectin protein for use as claimed in claim 6, wherein the acute inflammation is caused by an allergic reaction, an infection or trauma.

8. The lectin protein for use as claimed in any preceding claim, wherein said treatment comprises curing the inflammation or the chronic inflammatory disease, or reducing symptoms associated with the inflammation.

9. The lectin protein for use as claimed in any preceding claim, wherein said prevention comprises preventing the onset of the inflammation, or preventing or slowing the progression of the chronic inflammatory disease.

10. The lectin protein for use as claimed in any preceding claim, wherein said treatment or prevention comprises administering the lectin protein at a dose of 1 to 50 mg/kg.

11. A pharmaceutical composition comprising a lectin protein and a pharmaceutically acceptable diluent or excipient, wherein the lectin protein is recombinant, is derived from *Sclerotium rolfsii,* and comprises an amino acid sequence of SEQ ID NO:2 for use in a method of treatment or prevention of inflammation in a subject, the method comprising administering the pharmaceutical composition to the subject.

12. The pharmaceutical composition for use as claimed in claim 11, wherein the inflammation is an inflammatory disease.

13. The pharmaceutical composition for use as claimed in claim 12, wherein the inflammatory disease is arthritis.

14. The pharmaceutical composition for use as claimed in one of claims 11 to 13, wherein the method comprises administering the lectin protein at a dose of 1 to 50 mg/kg.


**Patentansprüche**

1. Lektinprotein zur Behandlung oder Vorbeugung von Entzündungen bei einem Patienten, der dies benötigt, wobei das Lektinprotein rekombinant ist, aus *Sclerotium rolfsii* gewonnen wird und eine Aminosäuresequenz von SEQ ID NO:2 umfasst.

2. Lektinprotein zur Verwendung nach einem vorstehenden Anspruch, wobei die Behandlung oder Vorbeugung von Entzündungen a) die Hemmung der Sekretion von entzündungsfördernden Zytokinen; und/oder b) die Senkung der Spiegel von C-reaktivem Protein (CRP) im Blut; und/oder c) die Stimulierung der ALP-Aktivität und/oder von Kollagenspiegeln umfasst.

3. Lektinprotein zur Verwendung nach einem vorstehenden Anspruch, wobei der Patient an einer chronischen Entzündungskrankheit leidet.

4. Lektinprotein zur Verwendung nach Anspruch 3, wobei es sich bei der chronischen Entzündungskrankheit um Psoriasis, Psoriasis-Arthritis, rheumatoide Arthritis (RA), Hepatitis, Asthma, entzündliche Darmerkrankungen (einschließlich Morbus Crohn, Colitis ulcerosa und Reizdarmsyndrom (IBS)), Arteriitis temporalis, atopische Dermatitis, systemischen Lupus, Multiple Sklerose oder Sarkoidose handelt.

5. Lektinprotein zur Verwendung nach Anspruch 4, wobei es sich bei der chronischen Entzündungskrankheit um rheumatoide Arthritis (RA) handelt.

6. Lektinprotein zur Verwendung nach einem der Ansprüche 1 bis 2, wobei der Patient an einer akuten Entzündung leidet.

7. Lektinprotein zur Verwendung nach Anspruch 6, wobei die akute Entzündung durch eine allergische Reaktion, eine Infektion oder ein Trauma verursacht wird.

8. Lektinprotein zur Verwendung nach einem vorstehenden Anspruch, wobei die Behandlung die Heilung der Entzündung oder der chronischen Entzündungskrankheit oder die Linderung der mit der Entzündung verbundenen Symptome umfasst.

9. Lektinprotein zur Verwendung nach einem vorstehenden Anspruch, wobei die Vorbeugung Vorbeugung des Beginns der Entzündung oder Vorbeugung oder Verlangsamen des Fortschreitens der chronischen Entzündungskrankheit umfasst.

10. Lektinprotein zur Verwendung nach einem vorstehenden Anspruch, wobei die Behandlung oder Vorbeugung die Verabreichung des Lektinproteins in einer Dosis von 1 bis 50 mg/kg umfasst.

11. Pharmazeutische Zusammensetzung, umfassend ein Lektinprotein und ein pharmazeutisch akzeptables Verdünnungsmittel oder einen Hilfsstoff, wobei das Lektinprotein rekombinant ist, aus *Sclerotium rolfsii* gewonnen wird und eine Aminosäuresequenz von SEQ ID NO:2 umfasst, zur Verwendung in einem Verfahren zur Behandlung oder Vorbeugung von Entzündungen bei einem Patienten, wobei das Verfahren die Verabreichung der pharmazeutischen Zusammensetzung an den Patienten umfasst.

12. Pharmazeutische Zusammensetzung zur Verwendung nach Anspruch 11, wobei es sich bei der Entzündung um eine Entzündungskrankheit handelt.

13. Pharmazeutische Zusammensetzung zur Verwendung nach Anspruch 12, wobei es sich bei der Entzündungskrankheit um Arthritis handelt.

14. Pharmazeutische Zusammensetzung zur Verwendung nach einem der Ansprüche 11 bis 13, wobei das Verfahren die Verabreichung des Lektinproteins in einer Dosis von 1 bis 50 mg/kg umfasst.

**Revendications**

1. Protéine de lectine pour utilisation dans le traitement ou la prévention d'une inflammation chez un sujet qui en a besoin, dans laquelle la protéine de lectine est recombinante, est dérivée de *Sclerotium rolfsii,* et comprend une séquence d'acides aminés SEQ ID NO:2.

2. Protéine de lectine pour utilisation selon une quelconque revendication précédente, dans laquelle le traitement ou la prévention d'une inflammation comprend a) l'inhibition de la sécrétion de cytokines inflammatoires ; et/ou b) la réduction des niveaux de protéine (-réactive (CRP) dans le sang ; et/ou c) la stimulation des niveaux de l'activité ALP et/ou de collagène.

3. Protéine de lectine pour utilisation selon une quelconque revendication précédente, dans laquelle le sujet souffre d'une maladie inflammatoire chronique.

4. Protéine de lectine pour utilisation selon la revendication 3, dans laquelle la maladie inflammatoire chronique est le psoriasis, l'arthrite psoriasique, la polyarthrite rhumatoïde (PR), l'hépatite, l'asthme, les maladies inflammatoires de l'intestin (y compris la maladie de Crohn, la colite et le syndrome de l'intestin irritable (SII)), l'artérite temporale, la dermatite atopique, le lupus systémique, la sclérose en plaques ou la sarcoïdose.

5. Protéine de lectine pour utilisation selon la revendication 4, dans laquelle la maladie inflammatoire chronique est l'arthrite rhumatoïde (AR).

6. Protéine de lectine pour utilisation selon l'une des revendications 1 à 2, dans laquelle le sujet présente une inflammation aiguë.

7. Protéine de lectine pour utilisation selon la revendication 6, dans laquelle l'inflammation aiguë est causée par une réaction allergique, une infection ou un traumatisme.

8. Protéine de lectine pour utilisation selon une quelconque revendication précédente, dans laquelle ledit traitement consiste à guérir l'inflammation ou la maladie inflammatoire chronique, ou à réduire les symptômes associés à l'inflammation.

9. Protéine de lectine pour utilisation selon une quelconque revendication précédente, dans laquelle ladite prévention consiste à prévenir l'apparition de l'inflammation, ou à prévenir ou ralentir la progression de la maladie inflammatoire chronique.

10. Protéine de lectine pour utilisation selon une quelconque revendication précédente, dans laquelle ledit traitement ou ladite prévention consiste à administrer la protéine de lectine à une dose de 1 à 50 mg/kg.

11. Composition pharmaceutique comprenant une protéine de lectine et un diluant ou excipient pharmaceutiquement acceptable, dans laquelle la protéine de lectine est recombinante, est dérivée de *Sclerotium rolfsii,* et comprend une séquence d'acides aminés de SEQ **ID** NO:2 pour une utilisation dans un procédé de traitement ou de prévention d'une inflammation chez un sujet, le procédé comprenant l'administration de la composition pharmaceutique au sujet.

12. Composition pharmaceutique pour utilisation selon la revendication 11, dans laquelle l'inflammation est une maladie inflammatoire.

13. Composition pharmaceutique pour utilisation selon la revendication 12, dans laquelle la maladie inflammatoire est l'arthrite.

14. Composition pharmaceutique pour utilisation selon l'une des revendications 11 à 13, dans laquelle le procédé comprend l'administration de la protéine de lectine à une dose de 1 à 50 mg/kg.

**Figure 1**

**Figure 2**

← → Inflammatory edema        Knee Joint: Cartilage Bone damage        ( Ankle Joint: Angle

● Ankle Joint bone destruction

**Figure 3**

**Figure 4**

**EP 3 849 586 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 201821009667
- WO 2010050369 A1
- GB 2270626 A
- WO 0057897 A1
- WO 2010095143 A2
- WO 2010095143 A
- WO 2014203261 A

**Non-patent literature cited in the description**

- **ALTSCHUL et al.** *Nucleic Acids Res.*, 01 September 1997, vol. 25 (17), 3389-402